(19) Europäisches Patentamt European Patent Office Office européen des brevets

(11) **EP 4 776 221 A1**

(12) **EUROPEAN PATENT APPLICATION**

(43) Date of publication:
15.07.2026 Bulletin 2026/29

(21) Application number: 26150822.0

(22) Date of filing: 08.01.2026

(51) International Patent Classification (IPC):
**G06T 7/00** (2017.01)     **A61B 8/00** (2006.01)

(52) Cooperative Patent Classification (CPC):
**G06T 7/0012; A61B 8/483;** G06T 2207/10016;
G06T 2207/10132; G06T 2207/10136;
G06T 2207/20081; G06T 2207/20084;
G06T 2207/30096

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 08.01.2025  CN 202510028484
20.11.2025  JP 2025200912

(71) Applicant: **Canon Medical Systems Corporation**
**Tochigi 324- (JP)**

(72) Inventors:
• **GOU, Panjie**
  **Beijing (CN)**
• **HAN, Bing**
  **Beijing (CN)**
• **ZHAO, Shun**
  **Beijing (CN)**

(74) Representative: **Marks & Clerk LLP**
**15 Fetter Lane**
**London EC4A 1BW (GB)**

(54) **MEDICAL IMAGE PROCESSING APPARATUS, ULTRASOUND DIAGNOSTIC APPARATUS, MEDICAL IMAGE PROCESSING METHOD, AND COMPUTER READABLE MEDIUM**

(57)    A medical image processing apparatus (102) according to an embodiment includes an image acquisition unit (112); a spatial information acquisition unit (152); a feature map generation unit (122, 132); and an execution unit (162). The image acquisition unit (112) is configured to acquire a plurality of medical images. The spatial information acquisition unit (152) is configured to acquire three-dimensional spatial information corresponding to the medical images and representing three-dimensional spatial characteristics. The feature map generation unit (122, 132) is configured to generate a feature map based on the medical images and the three-dimensional spatial information. The execution unit (162) is configured to execute processing on a target medical image based on the feature map.

FIG.1

IMAGE PROCESSING APPARATUS — 100

IMAGE ACQUISITION UNIT — 110

THREE-DIMENSIONAL FEATURE MAP GENERATION UNIT — 120

TWO-DIMENSIONAL FEATURE MAP GENERATION UNIT — 130

OUTPUT UNIT — 140

EP 4 776 221 A1

**Description**

FIELD

Embodiments disclosed herein and in the drawings relate to a medical image processing apparatus, an ultrasound diagnostic apparatus, a medical image processing method, and a computer readable medium.

BACKGROUND

Conventionally, lesion detection based on video is a localized examination means for the organ under inspection, and multiple types of lesions may appear in the video images. Common medical video examinations include colonoscopy, gastroscopy, endoscopic ultrasonography, and external ultrasound. The ultrasound lesion detection technology can assist physicians in identifying regions potentially indicative of lesions in ultrasound images in a timely manner, thereby increasing the detection rate of disease during routine examinations.

In detection tasks using video, it is difficult to accurately determine the type and location of a lesion with only one frame image. In medical video detection, features of each frame image in a time-series image are usually extracted first, and the features of the time-series image are then acquired through a feature fusion method to more accurately determine the location and type of lesion. The feature fusion technique includes, for example, a method based on feature aggregation, such as optical flow feature mapping.

As an example, a target detection method includes: acquiring a current frame and a preceding key frame corresponding to the current frame from a video frame sequence; determining an optical flow feature map and an optical flow map between the preceding key frame and the current frame; upon determining that, based on the optical flow map, the current frame is a non-key frame, acquiring a key frame feature corresponding to the preceding key frame; obtaining an image feature corresponding to the current frame by performing an affine transformation on the key frame feature based on the optical flow map; detecting pixel levels of individual pixel points in the current frame in accordance with the image feature; and obtaining a detection result corresponding to a target in the current frame. However, in this method, although the key frame feature is overlaid onto the current frame using the optical flow feature mapping, the extracted and overlaid feature remains as a two-dimensional feature, resulting in the loss of spatial characteristics of a three-dimensional tissue structure.

Another example includes a method of expanding an effective detection range by utilizing the panoramic image stitching technology. In this method, images collected at previous time points are stored, and a wide-range image is acquired using the image stitching technology to expand the perceptual range of the detection model and improve detection accuracy. However, in this method, although two-dimensional images are stitched in a case of using the previous frame, an extracted image feature remains as a two-dimensional surface feature, resulting in the loss of spatial characteristics of a three-dimensional tissue structure.

SUMMARY OF INVENTION

A medical image processing apparatus comprising:

an image acquisition unit configured to acquire a plurality of medical images;
a spatial information acquisition unit configured to acquire three-dimensional spatial information corresponding to the medical images and representing three-dimensional spatial characteristics;
a feature map generation unit configured to generate a feature map based on the medical images and the three-dimensional spatial information; and
an execution unit configured to execute processing on a target medical image based on the feature map.

The feature map generation unit may be further configured to generate a three-dimensional feature map based on the medical images and the three-dimensional spatial information, and generate a two-dimensional feature map by compressing the three-dimensional feature map, and
the execution unit may be further configured to execute processing on the target medical image based on the two-dimensional feature map.

The image acquisition unit may be further configured to acquire a plurality of medical images in a time series, and the feature map generation unit may be further configured to generate the two-dimensional feature map by compressing the three-dimensional feature map in a time-series direction.

The feature map generation unit may be further configured to construct a three-dimensional image by aligning the medical images in a chronological order, and generate the three-dimensional feature map based on the three-dimensional image.

The feature map generation unit may be further configured to construct the three-dimensional image using smaller than a predetermined number of images among the medical images.

The spatial information acquisition unit may be further configured to acquire, as the three-dimensional spatial information, a similarity between images included in the medical images, and the feature map generation unit may be further configured to construct the three-dimensional image using a medical image whose similarity is smaller than a threshold value, among the medical images.

The spatial information acquisition unit may be further configured to acquire, as the similarity, a value representing an error between the images included in the medical images, and the feature map generation unit may be further configured to construct the three-dimensional image using, as the medical image whose similarity is smaller than a threshold value, a medical image whose value representing the error between the images is greater than a threshold value.

The spatial information acquisition unit may be further configured to acquire, as the similarity, a value representing a distance between the images included in the medical images based on spatial coordinates corresponding to each of the medical images, and the feature map generation unit may be further configured to construct the three-dimensional image using, as the medical image whose similarity is less than a threshold value, a medical image whose value representing the distance between the images is greater than a threshold value.

The feature map generation unit may be further configured to generate the three-dimensional feature map by applying three-dimensional convolution to the three-dimensional image using a convolutional neural network model.

The feature map generation unit may be further configured to compress the three-dimensional feature map in the time-series direction by averaging the three-dimensional feature map in the time-series direction.

The feature map generation unit may be further configured to assign weights to the three-dimensional feature map at individual positions in the time-series direction, and compress the three-dimensional feature map in accordance with the assigned weight to generate the two-dimensional feature map.

The feature map generation unit may be further configured to assign greater weights to positions closer to the current frame in the time-series direction with respect to the three-dimensional feature map.

The feature map generation unit may be further configured to use, as the weights, weights obtained by training a machine learning model in which the weights set for the individual positions in the time-series direction are used as learning parameters.

The execution unit may be further configured to execute, as the processing on the target medical image, a target extraction process of detecting a target object in the target medical image.

The execution unit may be further configured to execute, as the processing on the target medical image, an image segmentation process of performing image segmentation on the target medical image.

The execution unit may be further configured to execute, as the processing on the target medical image, an image classification process of determining a type of the target medical image.

An ultrasound diagnostic apparatus comprising:

an ultrasound probe capable of imaging a subject; and
processing circuitry, wherein
the processing circuitry includes

an image acquisition unit configured to acquire a plurality of ultrasound medical images in a time series sequentially imaged by the ultrasound probe,
a spatial information acquisition unit configured to acquire three-dimensional spatial information corresponding to the ultrasound medical images and representing three-dimensional spatial characteristics,
a feature map generation unit configured to generate a feature map based on the ultrasound medical images and the three-dimensional spatial information, and
an execution unit configured to execute processing on a target ultrasound medical image based on the feature map.

A medical image processing method comprising:

acquiring a plurality of medical images;
acquiring three-dimensional spatial information corresponding to the medical images and representing three-dimensional spatial characteristics;
generating a feature map based on the medical images and the three-dimensional spatial information; and
executing processing on a target medical image based on the feature map.

A computer readable medium comprising instructions that cause a computer to execute:

acquiring a plurality of medical images;
acquiring three-dimensional spatial information corresponding to the medical images and representing three-dimensional spatial characteristics;
generating a feature map based on the medical images and the three-dimensional spatial information; and
executing processing on a target medical image based on the feature map.

BRIEF DESCRIPTION OF THE DRAWINGS

FIG. 1 is a block diagram illustrating a functional configuration of an image processing apparatus according to a first embodiment;
FIG. 2 is a schematic diagram illustrating the generation of a three-dimensional feature map from a three-dimensional image in the first embodiment;
FIG. 3 is a schematic diagram illustrating the generation of a two-dimensional feature map by compressing the three-dimensional feature map in the first embodiment;
FIG. 4 is a flowchart illustrating an image processing method executed by the image processing apparatus according to the first embodiment;
FIG. 5 is a block diagram illustrating a functional configuration of an image processing apparatus according to a second embodiment;
FIG. 6 is a block diagram illustrating a functional configuration of an image selection unit according to the second embodiment;
FIG. 7 is a diagram illustrating a flowchart executed when an image is selected in real time in the second embodiment;
FIG. 8 is a flowchart illustrating one specific example at step S113 of FIG. 7;
FIG. 9 is a block diagram illustrating a functional configuration of an image processing apparatus according to a third embodiment;
FIG. 10 is a block diagram illustrating a functional configuration of a detection unit according to the third embodiment;
FIG. 11 is a block diagram illustrating a functional configuration of an ultrasound diagnostic apparatus using the image processing apparatus according to the third embodiment;
FIG. 12 is a timing chart illustrating operation timings of respective components in the ultrasound diagnostic apparatus using the image processing apparatus according to the third embodiment;
FIG. 13 is a diagram illustrating an example of a detection result of lesion detection performed by the image processing apparatus 102 according to the third embodiment;
FIG. 14 is a diagram illustrating an example of a detection result of lesion detection performed by the image processing apparatus 102 according to the third embodiment; and
FIG. 15 is a block diagram illustrating a functional configuration of an image processing apparatus according to a fourth embodiment.

DETAILED DESCRIPTION

A medical image processing apparatus according to an embodiment includes an image acquisition unit; a spatial information acquisition unit; a feature map generation unit; and an execution unit. The image acquisition unit is configured to acquire a plurality of medical images. The spatial information acquisition unit is configured to acquire three-dimensional spatial information corresponding to the medical images and representing three-dimensional spatial characteristics. The feature map generation unit is configured to generate a feature map based on the medical images and the three-dimensional spatial information. The execution unit is configured to execute processing on a target medical image based on the feature map.

An image processing apparatus according to one embodiment includes: an image acquisition unit configured to acquire a plurality of images; a three-dimensional feature map generation unit configured to generate a three-dimensional feature map corresponding to a three-dimensional image constructed, as a single three-dimensional image, from the images; a two-dimensional feature map generation unit configured to generate a two-dimensional feature map by compressing the three-dimensional feature map; and an output unit configured to output the two-dimensional feature map.

Therefore, the two-dimensional feature map that reflects the spatial characteristics of the three-dimensional structure of a detection target can be output.

In the image processing apparatus, it is preferable that the three-dimensional feature map generation unit generates the three-dimensional feature map corresponding to the three-dimensional image configured in a time-series direction, and the two-dimensional feature map generation unit generates the two-dimensional feature map by compressing the three-dimensional feature map in a time-series direction.

Therefore, the two-dimensional feature map that reflects the spatial characteristics of the three-dimensional structure of a detection target can be output, while ensuring real-time detection.

In the image processing apparatus, it is preferable that the two-dimensional feature map generation unit assigns time series weights to the three-dimensional feature map in the time series direction and compresses the three-dimensional feature map with the time series weights into the two-dimensional feature map.

Therefore, with consideration given to the contribution levels of frames at a plurality of time points, the two-dimensional feature map that reflects the spatial characteristics of the three-dimensional structure of the detection target can be output.

In the image processing apparatus, it is preferable that higher time-series weights are assigned to regions closer to the current frame in the time-series direction in the three-dimensional feature map.

Therefore, with consideration given to the higher contribution level of regions closer to the current frame among frames at the time points, the two-dimensional feature map that reflects the spatial characteristics of the three-dimensional structure of the detection target can be output.

In the image processing apparatus, it is preferable that the time-series weights are learnable time-series weights trained through machine learning.

This enables machine learning to learn reasonable time series weights in the training process.

In the image processing apparatus, it is preferable that the number of images constituting the three-dimensional image is equal to or smaller than a predetermined number.

Therefore, by forming the three-dimensional image with a finite number of images, the computational complexity can be reduced, and the real-time capability of the output two-dimensional feature map can be improved.

It is preferable that the image processing apparatus further includes an image selection unit that selects an image from the images in a time series, and the three-dimensional feature map generation unit generates a three-dimensional feature map corresponding to a three-dimensional image constructed, as a single three-dimensional image, from a plurality of images selected by the image selection unit.

Therefore, the images that are more suitable for reflecting the spatial characteristics of the three-dimensional structure of the detection target can be selected.

In the image processing apparatus, it is preferable that the image selection unit calculates an image similarity between a current frame image and a previous frame image, and selects the current frame image in a case where the image similarity is equal to or smaller than a threshold value, and the three-dimensional feature map generation unit allows the current frame image selected by the image selection unit to be added into the images forming the three-dimensional image.

Therefore, the images that are more suitable for reflecting the spatial characteristics of the three-dimensional structure of the detection target can be selected when the image similarity is equal to or smaller than the threshold value.

It is preferable that the image processing apparatus further includes a spatial coordinate acquisition unit configured to acquire spatial coordinates corresponding to each of the images in a time series, in which the image selection unit calculates a coordinate similarity between a current frame image and a previous frame image based on the spatial coordinates, and selects the current frame image in a case where the coordinate similarity is equal to or smaller than a threshold value, and the three-dimensional feature map generation unit allows the current frame image selected by the image selection unit to be added into the images forming the three-dimensional image.

Therefore, the images that are more suitable for reflecting the spatial characteristics of the three-dimensional structure of the detection target can be selected when the spatial coordinate similarity is equal to or smaller than the threshold value.

In the image processing apparatus, it is preferable that the image selection unit calculates an image-to-image center distance and an inter-image angle between the current frame image and the previous frame image based on the spatial coordinates, and selects the current frame image in a case where the image-to-image center distance or the inter-image angle is greater than a threshold value.

Therefore, the images that are more suitable for reflecting the spatial characteristics of the three-dimensional structure of the detection target can be selected when the image-to-image center distance or the inter-image angle is greater than the threshold value.

In the image processing apparatus, it is preferable that the image acquisition unit acquires a plurality of ultrasound medical images imaged by an ultrasound probe, the three-dimensional feature map generation unit generates a three-dimensional feature map corresponding to a three-dimensional image constructed, as a single three-dimensional image, from the ultrasound medical images, the image processing apparatus further includes a detection unit configured to execute a detection task based on the two-dimensional feature map, and the output unit further outputs a detection result of the detection task.

Therefore, the two-dimensional feature map that reflects the spatial characteristics of the three-dimensional structure of the detection target in an ultrasound examination can be used for the detection task on the ultrasound medical images, resulting in an improvement in examination accuracy.

In the image processing apparatus, it is preferable that the image acquisition unit sequentially acquires a plurality of images, the three-dimensional feature map generation unit sequentially generates three-dimensional feature maps based on the sequentially acquired images, the two-dimensional feature map generation unit sequentially generates two-dimensional feature maps by compressing the sequentially generated three-dimensional feature maps, and the output unit sequentially outputs the two-dimensional feature maps.

Therefore, the images can be sequentially acquired one by one for an image collection task in real-time and output the corresponding two-dimensional feature map.

In the image processing apparatus, it is preferable that the image acquisition unit sequentially acquires a predetermined number of frame images.

Therefore, the two-dimensional feature maps corresponding to the predetermined number of frame images can be sequentially output.

It is preferable that the image processing apparatus further includes an image alignment unit that aligns a plurality of images in sequential order along a scanning direction, in which the three-dimensional feature map generation unit generates a three-dimensional feature map of a three-dimensional image constructed, as a single three-dimensional image, from the aligned images, and the two-dimensional feature map generation unit generates the two-dimensional feature map by compressing the three-dimensional feature map in the alignment direction.

Therefore, the two-dimensional feature map that reflects the spatial characteristics of the three-dimensional structure of a detection target can be output in accordance with the scanning direction such as the rotation of the probe, for example.

An image processing method according to one embodiment includes: an image acquiring step of acquiring a plurality of images; a three-dimensional feature map generating step of generating a three-dimensional feature map corresponding to a three-dimensional image constructed, as a single three-dimensional image, from the images; a two-dimensional feature map generating step of generating a two-dimensional feature map by compressing the three-dimensional feature map; and an output step of outputting the two-dimensional feature map.

Therefore, the two-dimensional feature map that reflects the spatial characteristics of the three-dimensional structure of a detection target can be output.

An ultrasound diagnostic apparatus according to one embodiment includes an ultrasound probe capable of moving and imaging a subject, a display, and an image processing unit, in which the image processing unit includes an image acquisition unit configured to acquire a plurality of ultrasound medical images in a time series imaged by the ultrasound probe, a three-dimensional feature map generation unit configured to generate a three-dimensional feature map corresponding to a three-dimensional image constructed, as a single three-dimensional image, from the ultrasound medical images, a two-dimensional feature map generation unit configured to generate a two-dimensional feature map by compressing the three-dimensional feature map, a detection unit configured to execute a detection task based on the two-dimensional feature map, and an output unit configured to output a detection result of the detection task, and the display displays at least one frame of the ultrasound medical image and the detection result of the detection task.

Therefore, the two-dimensional feature map that reflects the spatial characteristics of the three-dimensional structure of the detection target detected by ultrasound detection can be used for the detection task on the ultrasound medical images, resulting in an improvement in the detection accuracy of the ultrasound diagnostic apparatus.

A computer program product according to one embodiment includes a computer program that causes a computer to execute an image processing method, the method including: an image acquiring step of acquiring a plurality of images; a three-dimensional feature map generating step of generating a three-dimensional feature map corresponding to a three-dimensional image constructed, as a single three-dimensional image, from the images; a two-dimensional feature map generating step of generating a two-dimensional feature map by compressing the three-dimensional feature map; and an output step of outputting the two-dimensional feature map.

Therefore, the two-dimensional feature map that reflects the spatial characteristics of the three-dimensional structure of a detection target can be output.

Hereinafter, the specific embodiments disclosed in the present application will be described with reference to the drawings.

First Embodiment

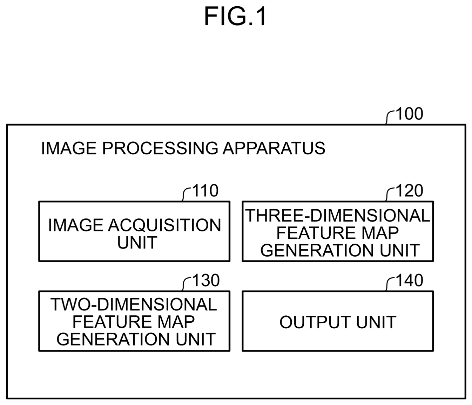

FIG. 1 is a block diagram illustrating a functional configuration of an image processing apparatus 100 according to a first embodiment. As illustrated in FIG. 1, the image processing apparatus 100 includes an image acquisition unit 110, a three-dimensional feature map generation unit 120, a two-dimensional feature map generation unit 130, and an output unit 140. The image processing apparatus 100 is an apparatus that obtains a two-dimensional feature map from an image queue formed of a plurality of images, and will be described in detail below. Here, the image processing apparatus 100 is an example of a medical image processing apparatus.

The image acquisition unit 110 acquires a plurality of medical images (hereinafter, referred to as images). Here, the image acquisition unit 110 is an example of an image acquisition unit.

Specifically, the image acquisition unit 110 acquires a plurality of medical images in a time series.

For example, the image acquisition unit 110 acquires the images from an image acquisition device. For example, the image acquisition unit 110 acquires a plurality of image frames within a certain time period from an ultrasound diagnostic apparatus via a wired or wireless communication connection. Examples of the ultrasound diagnostic apparatus include a B-mode ultrasonic diagnostic apparatus and a Doppler ultrasonic diagnostic apparatus. The image acquisition unit 110 may acquire medical images from another medical image acquisition device such as an endoscopic diagnosis device. The image acquisition unit 110 may acquire images from the other medical image acquisition device that is not a medical image acquisition device. The image acquisition unit 110 may acquire a plurality of images captured in advance, rather than acquiring images in real time. The image acquisition unit 110 may be an image acquisition device itself with external communication capabilities.

The imaging positions, angles, and the like of the images acquired by the image acquisition unit 110 vary in a three-dimensional space in accordance with the movement of a probe of the image acquisition device over a biological body such as a human in such a manner that a three-dimensional spatial structure of a detection target, such as tissue or an organ in the biological body such as a human, is reflected in the three-dimensional space.

In a case where the image acquisition device collects medical images in real time at a constant sampling frequency, the image acquisition unit 110 may acquire, as the images, several frames of images within a certain period of time, for example. In other words, the image acquisition frequency of the image acquisition unit 110 may be equal to the sampling frequency of the image acquisition device. In consideration of the computational capability for real-time computing, the image acquisition frequency of the image acquisition unit 110 may be lower than the sampling frequency of the image acquisition device, and for example, images may be acquired every few frames.

The three-dimensional feature map generation unit 120 and the two-dimensional feature map generation unit 130 generate feature maps based on the medical images acquired by the image acquisition unit 110. Here, the three-dimensional feature map generation unit 120 and the two-dimensional feature map generation unit 130 are examples of feature map generation units.

Specifically, the three-dimensional feature map generation unit 120 generates a three-dimensional feature map based on the medical images acquired by the image acquisition unit 110.

More specifically, the three-dimensional feature map generation unit 120 constructs a three-dimensional image by aligning the medical images acquired by the image acquisition unit 110 in chronological order, and generates a three-dimensional feature map based on the three-dimensional image.

In this case, for example, the three-dimensional feature map generation unit 120 constructs the three-dimensional image using smaller than a predetermined number of the medical images acquired by the image acquisition unit 110.

For example, the three-dimensional feature map generation unit 120 generates the three-dimensional feature map by applying three-dimensional convolution to the three-dimensional image using a convolutional neural network model.

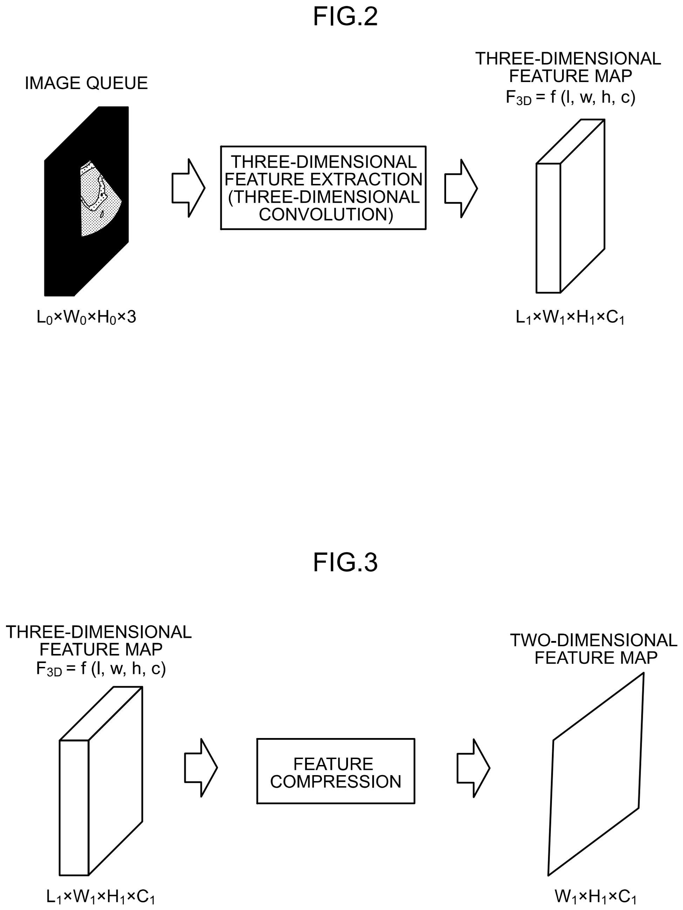

For example, the three-dimensional feature map generation unit 120 generates a three-dimensional feature map corresponding to a three-dimensional image (volume) formed, as a single three-dimensional image, from the images acquired by the image acquisition unit 110. FIG. 2 is a schematic diagram illustrating the generation of a three-dimensional feature map from a three-dimensional image in the first embodiment.

First, as illustrated in FIG. 2, the three-dimensional feature map generation unit 120 sequentially aligns the images acquired by the image acquisition unit 110 to construct a three-dimensional image as one image queue. For example, in a case where the image acquisition unit 110 acquires images in real time in accordance with the collection timing of the image acquisition device, the three-dimensional feature map generation unit 120 aligns the images in a time series into a single image queue. The alignment order of the images is not limited to the aforementioned order. For example, in a case where the image acquisition unit 110 acquires a plurality of images previously captured instead of acquiring images in real time, the images may be aligned in accordance with information such as an imaging angle.

The three-dimensional feature map generation unit 120 then controls the number of images forming the three-dimensional image to be equal to or smaller than a predetermined threshold value. For example, the number of frames of the images in a time series, constructing the three-dimensional image, is equal to or smaller than a predetermined number of frames. Therefore, by forming the three-dimensional image with a finite number of images, the computational complexity can be reduced, and the real-time capability can be improved.

As illustrated in FIG. 2, in a case where a resolution per image is defined as $W_0 \times H_0$, the number of images constituting the three-dimensional image is defined as $L_0$, and the number of RGB image channels is defined as 3, the size of the three-dimensional image is $L_0 \times W_0 \times H_0 \times 3$.

The three-dimensional feature map generation unit 120 generates a three-dimensional feature map by applying three-dimensional convolution to the three-dimensional image using a convolutional neural network (CNN) model. This three-dimensional image corresponds to an input layer of a convolutional neural network, and is input to the convolutional neural network model, and the convolutional neural network model performs a three-dimensional convolution operation on the input using a three-dimensional convolution operator as a convolution kernel to output a three-dimensional feature map. The convolutional neural network model may include one or more convolutional layers. The three-dimensional feature map is input to a subsequent convolutional layer as an output from the convolutional layer to generate a higher-level three-dimensional feature map. Each convolutional layer performs operations using one or more three-dimensional convolution operators, and the three-dimensional convolution operators may have different sizes. The convolutional neural network model may further include a pooling layer for reducing the dimensions of the output three-dimensional feature map.

In the convolutional neural network model, by executing convolution over spatial dimensions using the three-dimensional convolution operators, it is possible to preserve structural relationships in the space of the region to be detected, and as a result, the spatial correlation in the three-dimensional space of the images can be interpreted more effectively.

The details of the three-dimensional convolution executed in the convolutional neural network model are not limited thereto, and for example, parameters such as the size of the three-dimensional convolution operators and the step size of the convolution operations may be appropriately set according to the circumstances or computational capability. It is preferable to reduce the computational complexity by replacing two-dimensional convolution operators in a conventional two-dimensional real-time detection network with three-dimensional convolution operators. For example, a cube corresponding to the size of a two-dimensional convolution operator may be used as a convolution kernel.

A method for generating the three-dimensional feature map from the three-dimensional image is not limited thereto. For example, a neural network model other than CNN or another image feature extraction algorithm may be used.

As illustrated in FIG. 2, the three-dimensional feature map generated by the three-dimensional feature map generation unit 120 can be represented by $F_{3D} = f(l, w, h, c)$, and the size thereof is $L_1 \times W_1 \times H_1 \times C_1$. Here, $W_1$, $H_1$, and $L_1$ respectively denote the width, height, and length of the three-dimensional feature map, and $C_1$ denotes the number of channels of the three-dimensional feature map.

The two-dimensional feature map generation unit 130 generates a two-dimensional feature map by compressing the three-dimensional feature map generated by the three-dimensional feature map generation unit 120.

Specifically, the two-dimensional feature map generation unit 130 generates a two-dimensional feature map by compressing the three-dimensional feature map generated by the three-dimensional feature map generation unit 120, in a time-series direction.

For example, the two-dimensional feature map generation unit 130 compresses the three-dimensional feature map in the time-series direction by averaging the three-dimensional feature map, generated by the three-dimensional feature map generation unit 120, in the time-series direction.

FIG. 3 is a schematic diagram illustrating the generation of a two-dimensional feature map by compressing the three-dimensional feature map in the first embodiment.

As illustrated in FIG. 3, the two-dimensional feature map generation unit 130 compresses the three-dimensional feature map in a length ($L_1$) direction of the three-dimensional feature map, and the size of the compressed two-dimensional feature map is $W_1 \times H_1 \times C_1$. For example, in a case where the three-dimensional feature map generation unit 120 generates a three-dimensional feature map by aligning the images into a single image queue in a time series, the length direction of the three-dimensional feature map corresponds to the time-series direction, and the two-dimensional feature map generation unit 130 compresses the three-dimensional feature map in the time-series direction.

For example, as represented by Equation (1), the two-dimensional feature map generation unit 130 generates a two-dimensional feature map ($F_{2D}$) by averaging a three-dimensional feature map ($F_{3D}$) in the length ($L_1$) direction of the three-dimensional feature map.

$$F_{2D} = \frac{1}{L_1} \sum_{l \in (1, L_1)} f_{3D}(l, w, h, c) = f_{2D}(w, h, c) \qquad \cdots (1)$$

The compression method performed by the two-dimensional feature map generation unit 130 on the three-dimensional feature map is not limited to averaging, and other compression methods may also be employed.

The output unit 140 outputs the two-dimensional feature map generated by the two-dimensional feature map generation unit 130. Specifically, for example, the two-dimensional feature map is output to an external detection device, the external detection device executes a detection task based on the two-dimensional feature map. The detection task includes, but is not limited to, target detection, image segmentation, and image classification. The detection device performs, for example, a medical image detection task in real time based on the two-dimensional feature map output in real time from the output unit 140.

Since the two-dimensional feature map output from the output unit 140 is generated by compressing the three-dimensional feature map, the two-dimensional feature map includes three-dimensional features across the images, in other words, the three-dimensional structural relationships in space of the target region detected by imaging the images. Therefore, the image processing apparatus 100 can output the two-dimensional feature map that reflects the spatial characteristics of the three-dimensional structure of the detection target, and by using this two-dimensional feature map in the detection task, it is possible to improve detection accuracy, such as lesion detection accuracy in medical image detection.

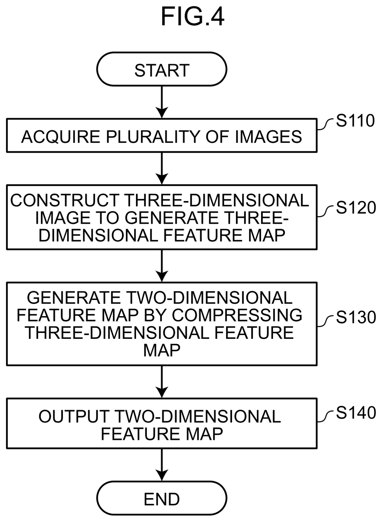

Hereinafter, the image processing method executed by the image processing apparatus 100 will be described with reference to FIG. 4. FIG. 4 is a flowchart illustrating an image processing method executed by the image processing apparatus 100 according to the first embodiment.

First, the image acquisition unit 110 acquires a plurality of images from the image acquisition device (S110). The three-dimensional feature map generation unit 120 then generates a three-dimensional feature map corresponding to a three-dimensional image constructed, as a single three-dimensional image, from the images acquired by the image acquisition unit 110 (S120). The two-dimensional feature map generation unit 130 then generates a two-dimensional feature map by compressing the three-dimensional feature map generated by the three-dimensional feature map generation unit 120 (S130). Finally, the output unit 140 outputs the two-dimensional feature map generated by the two-dimensional feature map generation unit 130 (S140). By using the above-described image processing method, it is possible to output the two-dimensional feature map that reflects the spatial characteristics of the three-dimensional structure of the detection target, and by using this two-dimensional feature map in the detection task, it is possible to improve detection accuracy, such as lesion detection accuracy in medical image detection.

Second Embodiment

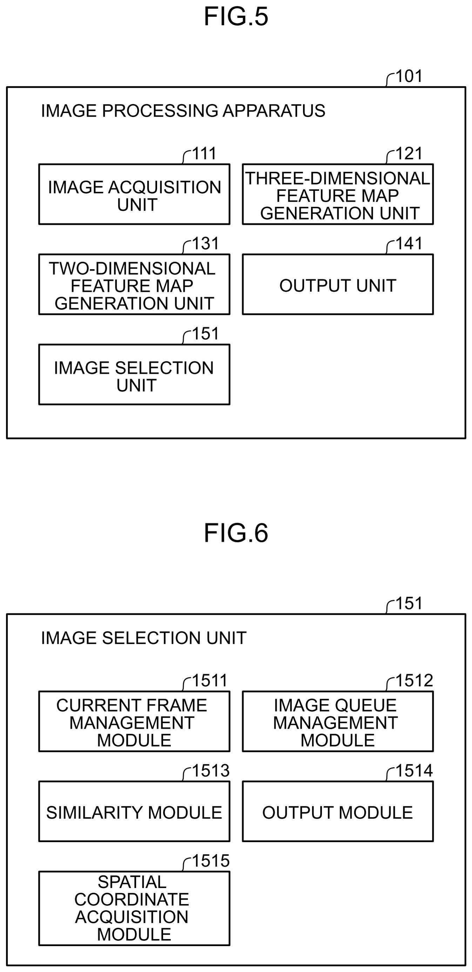

FIG. 5 is a block diagram illustrating a functional configuration of an image processing apparatus 101 according to a second embodiment. As illustrated in FIG. 5, an image processing apparatus 101 includes an image acquisition unit 111, a three-dimensional feature map generation unit 121, a two-dimensional feature map generation unit 131, an output unit 141, and an image selection unit 151. In the second embodiment, a specific case is considered in which the image processing apparatus 101 generates a two-dimensional feature map in real time while the probe of a medical image acquisition device moves in real time over the surface of the subject. Hereinafter, differences between the second embodiment and the first embodiment will be described. Here, the image processing apparatus 101 is an example of a medical image processing apparatus.

Medical images collected in real time by the probe of the medical image acquisition device are input to the image acquisition unit 111; in other words, during a single collection task of the medical image acquisition device, medical images are continuously input in a time-series manner.

The image selection unit 151 acquires three-dimensional spatial information corresponding to the medical images acquired by the image acquisition unit 111 and representing three-dimensional spatial characteristics. Here, the image selection unit 151 is an example of the spatial information acquisition unit.

Specifically, the image selection unit 151 acquires, as the three-dimensional spatial information, a similarity between images included in the medical images acquired by the image acquisition unit 111.

In this case, for example, the image selection unit 151 acquires, as the similarity, a value representing an error between images included in the medical images acquired by the image acquisition unit 111.

Alternatively, for example, the image selection unit 151 acquires, as the similarity, a value representing a distance between images included in the medical images, based on spatial coordinates corresponding to each of the medical images acquired by the image acquisition unit 111.

For example, the image selection unit 151 selects images from the images acquired by the image acquisition unit 111, and inputs the selected images to the three-dimensional feature map generation unit 121 as a single image queue. FIG. 6 is a block diagram illustrating a functional configuration of an image selection unit 151 according to the second embodiment. As illustrated in FIG. 6, the image selection unit 151 includes a current frame management module 1511, an image queue management module 1512, a similarity module 1513, an output module 1514, and a spatial coordinate acquisition module 1515.

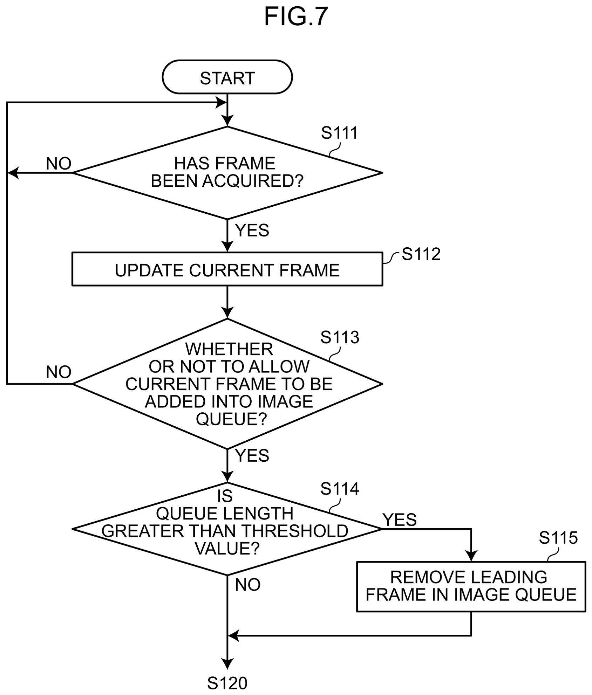

Hereinafter, with reference to FIG. 7, a specific description will be given of the processing flow executed in a case where images are selected by the image selection unit 151 from the images acquired by the image acquisition unit 111 and input to the three-dimensional feature map generation unit 121. FIG. 7 illustrates a flowchart executed when an image is selected in real time in the second embodiment.

First, in one collection task of the medical image acquisition device, the image acquisition unit 111 determines whether an image frame has been received from the medical image acquisition device (S111), and waits until the image frame is received (NO at S111).

In a case where the image acquisition unit 111 receives the image frame from the medical image acquisition device (YES at S111), the image acquisition unit 111 transmits the image frame to the current frame management module 1511 of the image selection unit 151. The current frame management module 1511 updates the current frame with the image frame (S112), and transmits the updated current frame to the similarity module 1513 of the image selection unit 151.

The similarity module 1513 of the image selection unit 151 determines whether or not to allow the current frame to be added into the image queue (S113). For example, the similarity module 1513 allows the first received current frame to be added into the image queue unconditionally, and subsequently determines whether or not to allow the updated current frame to be added into the image queue according to specific selection criteria. The specific selection criteria will be described below.

In a case where the similarity module 1513 determines not to allow the image frame to be added into the image queue (NO at S113), the image selection unit 151 waits until the current frame management module 1511 updates the current frame next time.

In a case of determining to allow the current frame to be added into the image queue (YES at S113), the similarity module 1513 causes the image queue management module 1512 to allow the current frame to be added into the image queue. Thereafter, the image queue management module 1512 determines whether or not the queue length of the updated image queue is greater than a threshold value (S114).

In a case of determining that the queue length of the updated image queue is not greater than the threshold value (NO at S114), the image queue management module 1512 outputs the updated image queue to the three-dimensional feature map generation unit 121 via the output module 1514, and causes the three-dimensional feature map generation unit 121 to generate a three-dimensional feature map corresponding to a three-dimensional image constructed, as a single three-dimensional image, from the image queue (S120).

In a case where the image queue management module 1512 determines that the queue length of the updated image queue is greater than the threshold value (YES at S114), the image queue management module 1512 removes the leading frame in the image queue (S115), and then outputs the newly updated image queue to the three-dimensional feature map generation unit 121 via the output module 1514. Here, the leading frame refers to the image frame that is the foremost (the oldest) image frame in the time-series direction in the image queue. The above-described threshold value related to the queue length is preferably 5 or greater and 15 or smaller, and more preferably 10. Therefore, by forming the three-dimensional image using an image queue with a predetermined threshold value or smaller, the computational complexity can be reduced, and the real-time capability can be improved.

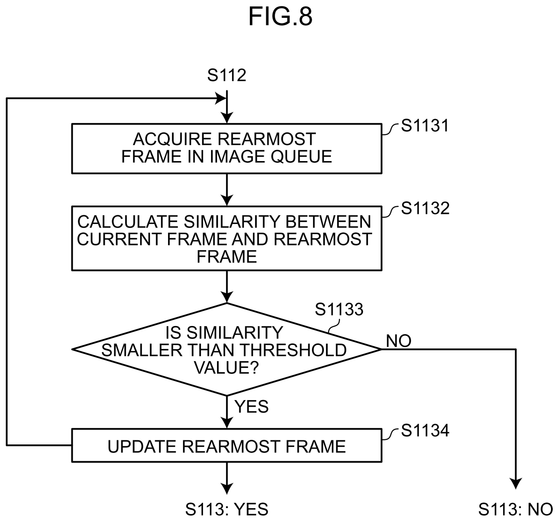

Hereinafter, with reference to FIG. 8, a specific description will be given of selection processing performed by the similarity module 1513 of the image selection unit 151. FIG. 8 is a flowchart illustrating one specific example at step S113 of FIG. 7.

When the current frame is updated at step S112, the similarity module 1513 first acquires the rearmost frame in the current image queue from the image queue management module 1512 (S1131). Here, the rearmost frame refers to the image frame that is the rearmost (the most recent) image frame in the time-series direction in the image queue.

Thereafter, the similarity module 1513 calculates the similarity between the current frame and the rearmost frame (S1132). The similarity may be at least one of image similarity or spatial coordinate similarity.

In a case of calculating image similarity, the similarity module 1513 uses, for example, the mean absolute difference (MAD) algorithm to calculate, as the similarity, an average value MAD of pixel-wise errors between the current frame and the rearmost frame in accordance with Equation (2).

$$\mathrm{MAD} = \frac{1}{W_0 \times H_0} \sum |I_c - I_{L0}| \qquad \cdots (2)$$

Here, $I_c$ is pixel data of the current frame and $I_{L0}$ is pixel data of the rearmost frame.

The method for calculating image similarity is not limited thereto, and for example, the image similarity may be evaluated using hash values.

In a case of calculating spatial coordinate similarity, the spatial coordinate acquisition module 1515 is first required to acquire spatial coordinates corresponding to each of the images in the time series from the medical image acquisition device. For example, the medical image acquisition device includes a positioning device such as a magnetic field coordinate transmitter in its probe, adds spatial coordinate information generated at the time of imaging the medical image to the header information of the image, and transmits the spatial coordinate information to the spatial coordinate acquisition module 1515. For example, the similarity module 1513 calculates an image center distance (ICD) using the spatial coordinate information, and calculates, as a similarity, a distance ICD between the center point coordinates of the current frame and the rearmost frame based on Equation (3).

$$\mathrm{ICD} = (C_C - C_{L0})^{\frac{1}{2}} \qquad \cdots (3)$$

Where $C_C$ denotes the coordinates of the center point of the current frame, and $C_{LO}$ denotes the coordinates of the center point of the rearmost frame.

The method for calculating a spatial coordinate similarity is not limited thereto, and for example, the similarity may be evaluated by calculating an angle between the plane normal vectors of the current frame and the rearmost frame. In other words, the similarity may be evaluated using the angle between images obtained by the medical image acquisition device. Both the distance between the center point coordinates and the angle between the images may be calculated.

In a case where the similarity is not calculated using the spatial coordinates, the image selection unit 151 does not need to include the spatial coordinate acquisition module 1515.

Thereafter, the similarity module 1513 determines whether or not the similarity between the current frame and the rearmost frame is smaller than a threshold value (S1133). Specifically, the similarity module 1513 determines that the similarity between the current frame and the rearmost frame is smaller than the threshold value in a case where at least one of the following conditions is satisfied: the calculated image similarity is smaller than the threshold value, or the spatial coordinate similarity is smaller than the threshold value. For example, in a case of calculating the similarity using the image similarity, the similarity module 1513 determines that the similarity between the current frame and the rearmost frame is smaller than the threshold value when the mean absolute difference (MAD) of the error between the current frame and the rearmost frame is greater than the threshold value. For example, in a case of calculating the similarity using the spatial coordinate similarity, the similarity module 1513 may determine that the similarity between the current frame and the rearmost frame is smaller than the threshold value when at least one of the distance between the center point coordinates and the angle between the images is greater than the threshold value.

In the case of determining that the similarity between the current frame and the rearmost frame is smaller than the threshold value (YES at S1133), the similarity module 1513 causes the image queue management module 1512 to allow the current frame to be added to the end of the image queue, thereby updating the rearmost frame (S1134). Thereafter, when the current frame is updated again at step S112, the similarity module 1513 retrieves the updated rearmost frame from the image queue management module 1512. When the image queue management module 1512 updates the rearmost frame (YES at S113), the process proceeds to the determination at step S114.

In a case where the similarity module 1513 determines that the similarity between the current frame and the rearmost frame is not smaller than the threshold value (NO at S1133), the current frame is not added to the image queue (NO at S113), and the process returns to step S111 to wait until the next image frame is acquired.

The similarity module 1513 may determine not only the similarity between the current frame and the rearmost frame, but also the similarity between the current frame and another frame or a plurality of other frames in the image queue.

In a case where the probe of the medical image acquisition device moves slowly or remains stationary, the similarity between the generated images is high. Thus, the image queue composed of such frames cannot reflect the actual spatial structure of the detection target. Therefore, since only images whose similarity is smaller than the threshold value are selected through the selection determination performed by the similarity module 1513 described above, a plurality of images each of which has a similarity greater than the threshold value and which are more suitable for reflecting the spatial characteristics of the three-dimensional structure of the detection target can be selected.

In the second embodiment, the three-dimensional feature map generation unit 121 and the two-dimensional feature map generation unit 131 generate feature maps based on the medical images acquired by the image acquisition unit 111 and the three-dimensional spatial information acquired by the image selection unit 151. Here, the three-dimensional feature map generation unit 121 and the two-dimensional feature map generation unit 131 are examples of feature map generation units.

The three-dimensional feature map generation unit 121 generates a three-dimensional feature map based on the medical images acquired by the image acquisition unit 111 and the three-dimensional spatial information acquired by the image selection unit 151.

Specifically, the three-dimensional feature map generation unit 120 constructs a three-dimensional image by aligning the medical images acquired by the image acquisition unit 110 in chronological order, and generates the three-dimensional feature map based on the three-dimensional image.

In this case, the three-dimensional feature map generation unit 121 constructs a three-dimensional image using medical images having a similarity acquired by the image selection unit 151 smaller than a threshold value, among the medical images acquired by the image acquisition unit 111.

For example, the three-dimensional feature map generation unit 121 constructs a three-dimensional image using medical images for which a value representing an error between images acquired by the image selection unit 151 is greater than a threshold value, as medical images having a similarity smaller than the threshold value.

Alternatively, for example, the three-dimensional feature map generation unit 121 constructs a three-dimensional image using medical images for which a value representing a distance between images acquired by the image selection unit 151 is greater than a threshold value, as medical images having a similarity smaller than the threshold value.

The three-dimensional feature map generation unit 121 constructs the selected image queue as a single three-dimensional image and generates a three-dimensional feature map based on three-dimensional convolution. Since this processing is the same as the processing performed by the three-dimensional feature map generation unit 120 in the first embodiment, a detailed description thereof will not be repeated.

The two-dimensional feature map generation unit 131 generates a two-dimensional feature map by compressing the three-dimensional feature map generated by the three-dimensional feature map generation unit 121.

Specifically, the two-dimensional feature map generation unit 131 assigns weights to individual positions in the time-series direction in the three-dimensional feature map generated by the three-dimensional feature map generation unit 121, and generates a two-dimensional feature map by compressing the three-dimensional feature map in accordance with the assigned weights.

In this case, for example, the two-dimensional feature map generation unit 131 assigns greater weights to positions closer to the current frame in the time-series direction with respect to the three-dimensional feature map generated by the three-dimensional feature map generation unit 121.

For example, the two-dimensional feature map generation unit 131 uses, as the weights, weights obtained by training a machine learning model in which the weights set for the individual positions in the time-series direction are used as learning parameters.

For example, as illustrated in Equation (4), the two-dimensional feature map generation unit 131 assigns a weight (l) serving as a time-series weight to a three-dimensional feature map ($F_{3D}$) generated by the three-dimensional feature map generation unit 121 in the time-series direction, and generates a two-dimensional feature map ($F_{2D}$) by compressing the weighted three-dimensional feature map in the time-series direction.

$$F_{2D} = \frac{1}{L_1} \sum_{l \in (1, L_1)} f_{3D}(l, w, h, c) \cdot weight(l) = f_{2D}(w, h, c) \qquad \cdots (4)$$

In the second embodiment, since it is expected that the two-dimensional feature map that reflects the spatial characteristics of the three-dimensional structure of the detection target is used for real-time detection of the current frame in the medical images, time-series weights are assigned when the three-dimensional feature map is compressed in the time-series direction, in consideration of the contribution level of each image in the image queue with respect to the detection task.

For example, since frames at time points closer to the current frame are expected to have a greater contribution level with respect to the detection task, higher time-series weights are assigned to regions closer to the current frame in the time-series direction. That is, the weight function weight (l) in Equation (4) is considered to be a monotonically increasing function. Therefore, the regions closer to the current frame in the three-dimensional feature map have higher time-series weights. For example, a weight function as illustrated in the following Equation (5) can be set.

$$weight(l) = (l - \delta)^\gamma \qquad \cdots (5)$$

The parameters $\delta$ and $\gamma$ herein may be set based on the practical experience.

It is also conceivable to train weight parameters (weight (1), weight (2), ..., weight ($L_1$)) learnable by a machine learning model. In other words, regarding the weight function weight (l), a machine learning model, such as a neural network, is used to learn a reasonable time-series weight during the training process. For example, the parameters $\delta$ and $\gamma$ in Equation (5) can be, for example, the learning parameters in a neural network.

Since the processing by the output unit 141 is the same as the processing in which the output unit 140 outputs the two-dimensional feature map in the first embodiment, a detailed description thereof will not be repeated.

As described above, the example has been exemplified in which the image selection unit 151 selects images from the images acquired by the image acquisition unit 111 when the probe of the medical image acquisition device is moved in real time over the surface of the subject. However, for example, in a case where the medical image acquisition device such as an ultrasound diagnostic apparatus performs a single real-time image collection task, the image processing apparatus 101 may sequentially acquire each frame image and sequentially output a two-dimensional feature map for each frame image. In other words, the image processing apparatus 101 may not include the image selection unit 151.

Specifically, in the image processing apparatus 101, at each time when the image acquisition unit 111 receives an image frame from the medical image acquisition device, the image acquisition unit 111 outputs a plurality of sequentially acquired images including the image frame to the three-dimensional feature map generation unit 121. The image acquisition unit 111 outputs the sequentially acquired images corresponding to a predetermined number of frames to the three-dimensional feature map generation unit 121.

Based on the sequentially acquired images from the image acquisition unit 111, the three-dimensional feature map generation unit 121 sequentially generates three-dimensional feature maps for individually acquired image frames by sequentially arranging the acquired images as a single image sequence constituting a three-dimensional image.

Similarly, the two-dimensional feature map generation unit 131 sequentially generates two-dimensional feature maps by compressing the three-dimensional feature maps sequentially generated by the three-dimensional feature map generation unit 121, and the output unit 141 sequentially outputs the two-dimensional feature maps.

Therefore, since the image acquisition unit 111 sequentially acquires the image frames one by one and the output unit 141 sequentially outputs the corresponding two-dimensional feature maps, the real-time capability and continuity of two-dimensional feature map generation are enhanced, thereby enabling support for real-time image collection tasks.

According to the image processing apparatus 101 of the second embodiment, it is possible to generate, in real time, a two-dimensional feature map reflecting spatial characteristics of the three-dimensional structure of a detection target when the probe of the medical image acquisition device moves over the surface of a subject, and it is possible to improve detection accuracy by using this two-dimensional feature map for the detection task on the medical image of the current frame.

Third Embodiment

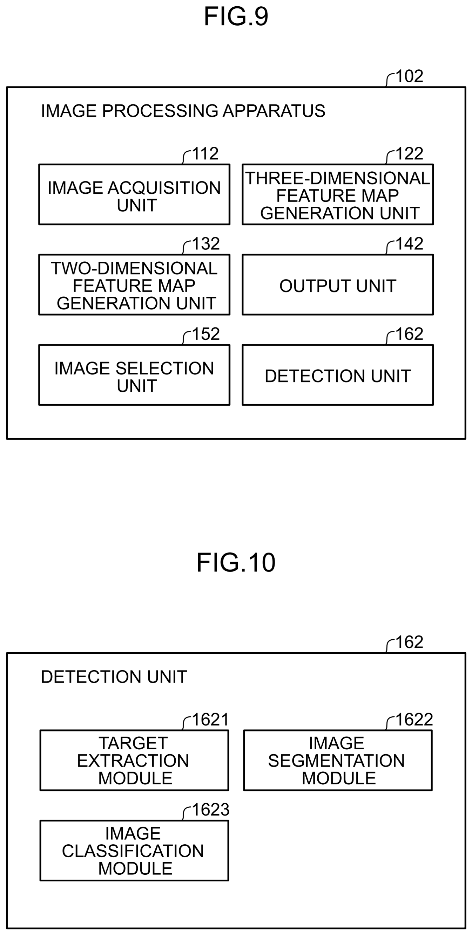

FIG. 9 is a block diagram illustrating a functional configuration of the image processing apparatus 102 according to the second embodiment. As illustrated in FIG. 9, the image processing apparatus 102 includes an image acquisition unit 112, a three-dimensional feature map generation unit 122, a two-dimensional feature map generation unit 132, an output unit 142, an image selection unit 152, and a detection unit 162. In the third embodiment, in addition to the second embodiment, a specific situation in which the image processing apparatus 102 further includes a function of executing a detection task on the medical images is considered. In particular, a case where the image processing apparatus 102 is used for ultrasound diagnosis is considered. Hereinafter, differences between the third embodiment and the second embodiment will be described. Here, the image processing apparatus 102 is an example of a medical image processing apparatus.

The processes executed by the image acquisition unit 112, the three-dimensional feature map generation unit 122, the two-dimensional feature map generation unit 132, and the image selection unit 152 in the third embodiment are similar to the processes executed by the image acquisition unit 111, the three-dimensional feature map generation unit 121, the two-dimensional feature map generation unit 131, and the image selection unit 151 in the second embodiment, and the two-dimensional feature map is generated in real time when the ultrasound probe moves over the surface of the subject in real time. Thus, a detailed description thereof will not be repeated.

The detection unit 162 executes processing on a target medical image based on the feature maps generated by the three-dimensional feature map generation unit 122 and the two-dimensional feature map generation unit 132. Here, the detection unit 162 is an example of an execution unit.

Specifically, the detection unit 162 executes processing on a target medical image based on the two-dimensional feature map generated by the two-dimensional feature map generation unit 132.

For example, the detection unit 162 executes a target extraction task of detecting a target object in the target medical image, as a process on the target medical image.

For example, the detection unit 162 executes an image segmentation task of performing image segmentation on the target medical image, as a process on the target medical image.

For example, the detection unit 162 executes an image classification task of determining the type of the target medical image, as a process on the target medical image.

FIG. 10 is a block diagram illustrating a functional configuration of the detection unit 162 according to the third embodiment. As illustrated in FIG. 10, the detection unit 162 includes a target extraction module 1621, an image segmentation module 1622, and an image classification module 1623.

The target extraction module 1621 detects whether or not a target object, such as a lesion or organ region, is present in the current frame (that is, the rearmost frame of the image queue before compression corresponding to the two-dimensional feature map), based on the two-dimensional feature map input from the two-dimensional feature map generation unit 132, and in a case where the target object is present, the target extraction module 1621 encloses the location of the target object with a prompt box and labels the type of the target object. That is, the target extraction module 1621 executes a target extraction task for ultrasound diagnosis.

For example, the target extraction module 1621 executes the target extraction task using a neural network model trained on several types of lesions, with the two-dimensional feature map as input. In the training process of this neural network model, training is performed based on loss functions between candidate boxes of target objects to be extracted and annotation boxes provided by physicians.

The image segmentation module 1622 performs image segmentation on the current frame (that is, the rearmost frame of the image queue before compression corresponding to the two-dimensional feature map) based on the two-dimensional feature map input from the two-dimensional feature map generation unit 132, and for example, the image segmentation module 1622 labels the pixel region of a target lesion or organ as foreground, and labels the remaining pixel regions as background. That is, the target extraction module 1621 executes an image segmentation task for ultrasound diagnosis.

For example, the image segmentation module 1622 executes an image segmentation task using a neural network model trained on several types of lesions and organs, with the two-dimensional feature map as input. In the training process of this neural network model, training is performed based on loss functions such as cross-entropy or Dice loss, for example.

The image classification module 1623 determines the type of the image of the current frame (that is, the rearmost frame of the image queue before compression corresponding to the two-dimensional feature map) based on the two-dimensional feature map input from the two-dimensional feature map generation unit 132, and the types of images refer to, for example, disease types or stages. That is, the target extraction module 1621 executes an image classification task for ultrasound diagnosis.

For example, the image classification module 1623 executes an image classification task using a neural network model trained on several types and stages of diseases, with the two-dimensional feature map as input. This neural network model corresponds to, for example, a fully connected layer in a convolutional neural network.

Since this neural network model takes the two-dimensional feature map as input, the neural network model similarly reflects the spatial characteristics of the three-dimensional structure of the detection target, while reducing the computational complexity and improving the feasibility of real-time detection, as compared to a case in which the detection task is executed using a three-dimensional feature map as input. It is also possible to utilize neural network models used in conventional two-dimensional real-time detection networks.

The detection unit 162 may selectively include one or more of the above-described modules according to detection requirements. The output unit 142 outputs the detection result of the detection unit 162.

The detection result of the detection unit 162 is output to an external display of the image processing apparatus 102 via the output unit 142. The image processing apparatus 102 may further include a display unit (not illustrated) configured to display an ultrasound image imaged by an ultrasound probe and perform labeling on the current frame of the ultrasound image using the detection result of the detection unit 162, and for example, a prompt box indicating the type and position of the detection target detected by the target extraction module 1621 is overlaid and displayed onto the ultrasound medical image of the current frame. The detection result may be output not only to a display device but also to a voice guidance device or the like.

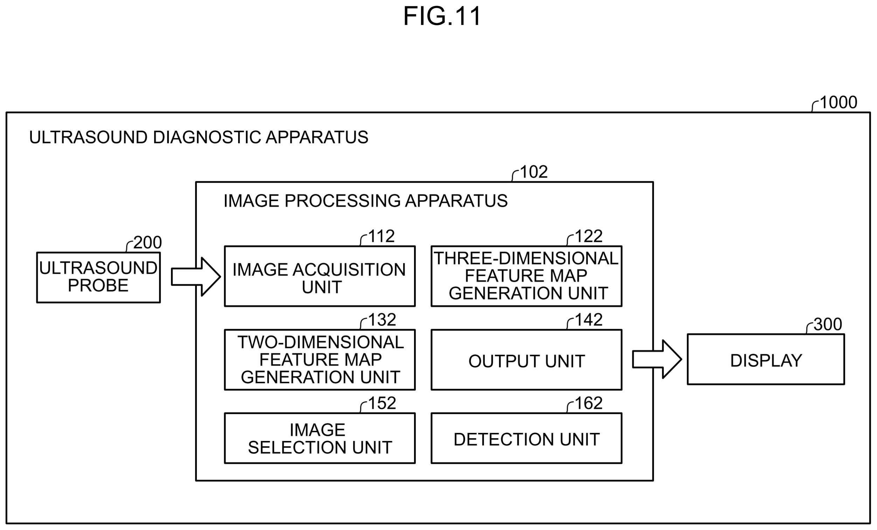

Hereinafter, an ultrasound diagnostic apparatus 1000 using the image processing apparatus 102 will be described. FIG. 11 is a block diagram illustrating a functional configuration of an ultrasound diagnostic apparatus 1000 using the image processing apparatus 102 according to the third embodiment. As illustrated in FIG. 11, the ultrasound diagnostic apparatus 1000 includes an ultrasound probe 200, an image processing apparatus 102, and a display 300. In the ultrasound diagnostic apparatus 1000, the image processing apparatus 102 functions as an image processing unit.

The ultrasound probe 200 is connected to the image processing apparatus 102 and the display 300 via wireless or wired communication, and transmits signals of images imaged in real time while the ultrasound probe 200 moves over the surface of a subject to the image processing apparatus 102 and the display 300. The display 300 receives the image signals from the ultrasound probe 200 and displays the images in real time. The image processing apparatus 102 receives image signals from the ultrasound probe 200, generates two-dimensional feature maps as described above, executes a detection task related to ultrasound diagnosis, and outputs the detection result to the display 300, and the display 300 performs labeling on the ultrasound image using the detection result.

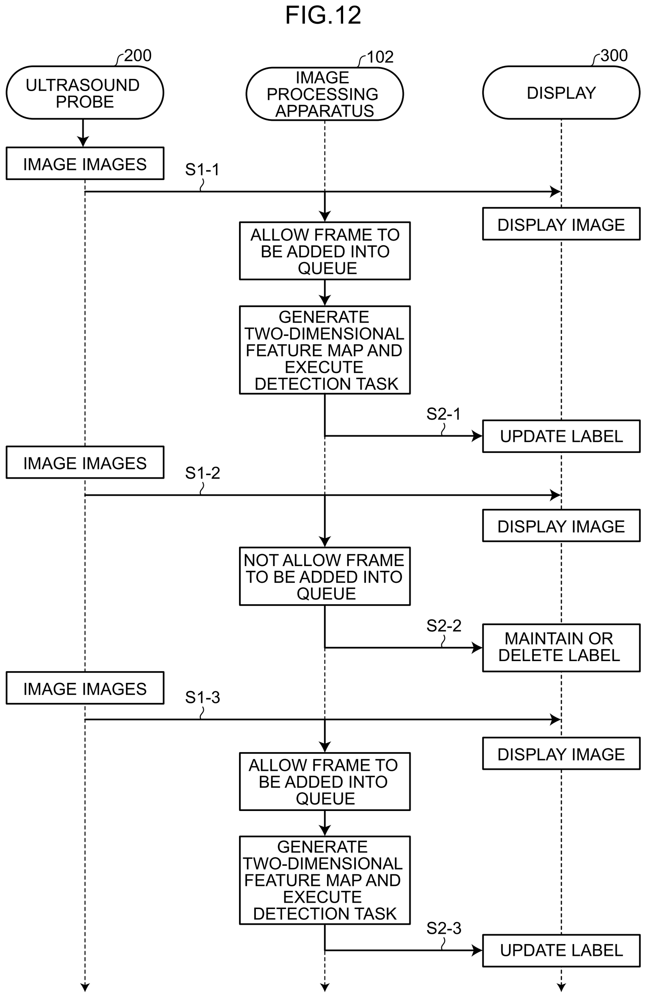

FIG. 12 is a timing chart illustrating operation timings of respective components in the ultrasound diagnostic apparatus 1000 using the image processing apparatus 102 according to the third embodiment. As illustrated in FIG. 12, when the ultrasound probe 200 images one frame of an ultrasound image, the image serving as the current frame is transmitted to the image processing apparatus 102 and the display 300 (S1-1), and the display 300 displays the image.

When receiving the current frame of the ultrasound image, the image processing apparatus 102 determines whether or not to allow the current frame to be added into the image queue. Specifically, the image selection unit 152 of the image processing apparatus 102 determines whether or not to allow the current frame to be added into the image queue, based on a determination criterion such as the similarity-based determination described in the second embodiment. In a case where it is determined that the current frame is added into the image queue, the image processing apparatus 102 treats the current frame as the rearmost frame of the image queue and generates a corresponding two-dimensional feature map. Then, the detection unit 162 of the image processing apparatus 102 executes a detection task on the current frame based on the two-dimensional feature map, and the output unit 142 transmits the detection result to the display 300 (S2-1).

When receiving the detection result, the display 300 displays at least one frame of the ultrasound medical image along with the detection result of the detection task. For example, the display 300 labels the detection result on the current frame. Specifically, the display 300 updates the previous label. This labeling may include, but is not limited to, a prompt box labeling the lesion region, labeling the pixels of the lesion region as foreground, or labeling the types and stages of disease.

Thereafter, the ultrasound probe 200 images another frame of an ultrasound image again, followed by repetition of the above-described data flow. For example, as illustrated in FIG. 12, after the ultrasound probe 200 transmits the current frame to the image processing apparatus 102 and the display 300 (S1-2), when the image processing apparatus 102 determines not to allow the current frame to be added into the image queue, neither the generation of a two-dimensional feature map nor the corresponding detection is performed. As a result, no data is transmitted to the display 300, and the image processing apparatus 102 either maintains the detection result labeled on the previous frame on the display 300 or transmits an instruction to the display 300 to delete the detection result labeled on the previous frame (S2-2).

The above-described data flow is repeatedly executed among the respective components of the ultrasound diagnostic apparatus 1000 during a single examination of the subject by the ultrasound diagnostic apparatus 1000, and for example, labeling is updated again at S1-3 to S2-3. Therefore, the detection results in the current frame are labeled in real time, and for example, real-time tracking of a target lesion or target organ is achieved.

For example, the ultrasound diagnostic apparatus 1000 is used for the detection tasks of a primary liver cancer (HCC), a metastatic liver cancer (Meta), a hepatic hemangioma (Hema), and a liver cyst (Cyst). As compared to detection based only on two-dimensional features, it is empirically confirmed that detection accuracy is improved by detecting the above-described types of lesions using a two-dimensional feature map that reflects the spatial characteristics of the three-dimensional structure of the detection target. Since the above-described types of lesions are detected based on the two-dimensional feature map, the detection speed can meet the demands of real-time detection tasks. For example, in a case where a commercially available graphics processor is used, the processing speed can meet the demands of real-time detection tasks.

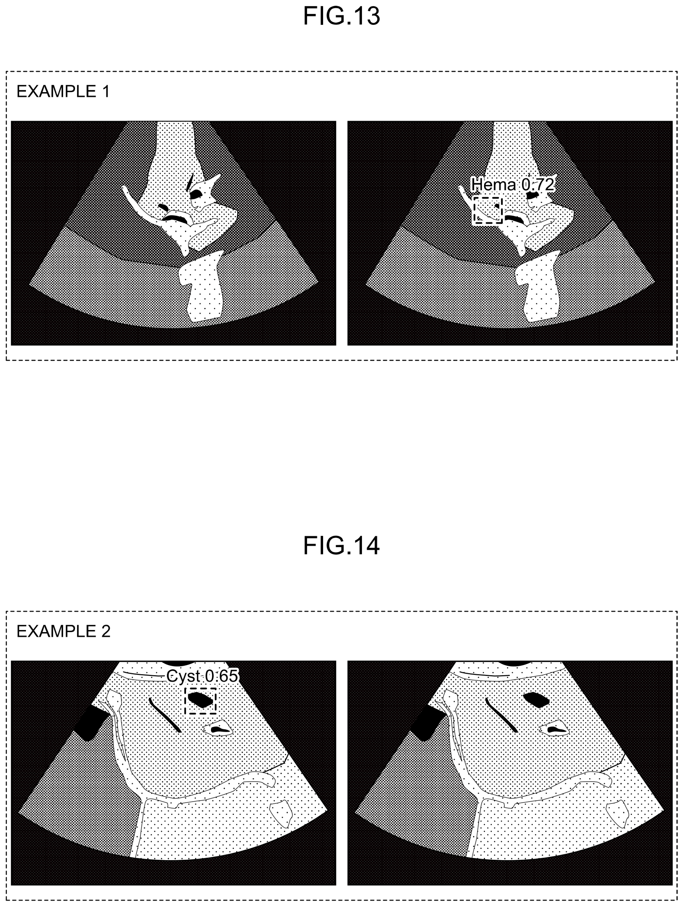

FIGS. 13 and 14 are diagrams illustrating examples of detection results of lesion detection performed by the image processing apparatus 102 according to the third embodiment.

In FIGS. 13 and 14, respectively, the image on the right side illustrates the detection result of lesion detection output by the above-described output unit 142, while the image on the left side illustrates, as a comparative example, the detection result obtained in a case where lesion detection is performed using a two-dimensional feature map that does not reflect the spatial characteristics of the three-dimensional structure of the detection target.

For example, as illustrated on the right side of FIG. 13, the output unit 142 overlays a prompt box, indicating the type and position of the detection target detected by the target extraction module 1621 of the detection unit 162, onto the current frame of the ultrasound medical image, and displays the image on the external display of the image processing apparatus 102 (for example, the display 300 of the ultrasound diagnostic apparatus 1000).

For example, as illustrated on the right side of FIG. 13, in a case where the lesion to be detected is a hemangioma (Hema), the output unit 142 overlays a rectangular prompt box onto an ultrasound medical image based on the processing result of the target extraction task executed by the target extraction module 1621 of the detection unit 162, the prompt box including a label "Hema" indicating the type of the detected hemangioma and a numeric value "0.72" indicating its location, and displays the image on the external display.

Here, for example, in a case where the lesion to be detected is a hemangioma (Hema), as illustrated on the left side of FIG. 13, the spatial characteristics of the three-dimensional tissue structure are lost in the comparative example, which may result in the hemangioma being missed in the detection. In contrast, as illustrated on the right side of FIG. 13, in the image processing apparatus 102 according to the third embodiment, since the lesion detection is performed using a feature map that reflects the spatial characteristics of the three-dimensional structure of the detection target, correct detection of the hemangioma can be achieved.

For example, in a case where the lesion to be detected is a cyst (Cyst), as illustrated on the left side of FIG. 14, the spatial characteristics of the three-dimensional tissue structure are lost in the comparative example, which may result in the gallbladder being erroneously detected as the cyst (Cyst). In contrast, as illustrated on the right side of FIG. 14, in the image processing apparatus 102 according to the third embodiment, since the lesion detection is performed using a feature map that reflects the spatial characteristics of the three-dimensional structure of the detection target, erroneous detection does not occur.

In the above-described third embodiment, the detection unit 162 is configured to execute processing on the target medical image based on the two-dimensional feature map generated by the two-dimensional feature map generation unit 132; however, the embodiment is not limited thereto.

For example, the detection unit 162 may execute processing on the target medical image based on a three-dimensional feature map generated by the three-dimensional feature map generation unit 122.

In this case, the target extraction module 1621 executes the target extraction task for ultrasound diagnosis based on the three-dimensional feature map input from the three-dimensional feature map generation unit 122. For example, the target extraction module 1621 executes the target extraction task using a neural network model trained several types of lesions, with the three-dimensional feature map as input. In the training process of this neural network model, training is performed based on loss functions between candidate boxes of target objects to be extracted and annotation boxes provided by physicians.

In this case, the image segmentation module 1622 executes the image segmentation task for ultrasound diagnosis based on the three-dimensional feature map input from the three-dimensional feature map generation unit 122. For example, the image segmentation module 1622 executes an image segmentation task using a neural network model trained on several types of lesions and organs, with the three-dimensional feature map as input. In the training process of this neural network model, training is performed based on loss functions such as cross-entropy or Dice loss, for example.

The image classification module 1623 executes the image classification task for ultrasound diagnosis based on the three-dimensional feature map input from the three-dimensional feature map generation unit 122. For example, the image classification module 1623 executes an image classification task using a neural network model trained on several types and stages of diseases, with the three-dimensional feature map as input. This neural network model corresponds to, for example, a fully connected layer in a convolutional neural network.

In a case where the detection unit 162 executes processing on the target medical image based on the three-dimensional feature map generated by the three-dimensional feature map generation unit 122, the image processing apparatus 102 may not include the two-dimensional feature map generation unit 132.

Fourth Embodiment

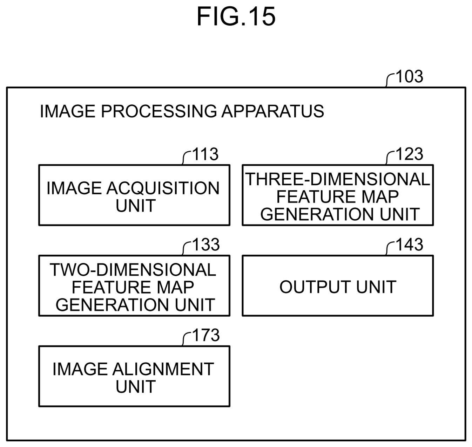

FIG. 15 is a block diagram illustrating a functional configuration of an image processing apparatus 103 according to a fourth embodiment. As illustrated in FIG. 15, the image processing apparatus 103 includes an image acquisition unit 113, a three-dimensional feature map generation unit 123, a two-dimensional feature map generation unit 133, an output unit 143, and an image alignment unit 173. The fourth embodiment differs from each of the above-described embodiments in that, while the above-described embodiments consider configuring an image queue in the time-series direction and compressing the three-dimensional feature map in the time-series direction, the fourth embodiment considers rearranging the images according to features other than the time-series. The differences of the fourth embodiment will be described below. Here, the image processing apparatus 103 is an example of a medical image processing apparatus.

The image alignment unit 173 aligns a plurality of images acquired by the image acquisition unit 113 and transmits an image queue formed of the aligned images to the three-dimensional feature map generation unit 123.

Specifically, the image alignment unit 173 aligns the images, for example, in the order of a scanning direction that is the normal vector of the imaging plane. For example, the image alignment unit 173 performs feature point matching on the images and calculates the normal vector of the imaging plane for each of the images. The image acquisition unit 113 also acquires the normal vector of the imaging plane corresponding to each of the images, for example, from a magnetic field coordinate transmitter or a gyroscope sensor built into a probe of an image acquisition device. The image alignment unit 173 aligns the images in the order in which the normal vectors of the imaging planes, when projected onto a predetermined plane, rotate in a clockwise direction, that is, in the order of rotation during probe scanning.

The three-dimensional feature map generation unit 123 generates a three-dimensional feature map corresponding to a three-dimensional image constructed, as a single three-dimensional image, from the aligned images. The two-dimensional feature map generation unit 133 generates a two-dimensional feature map by compressing the three-dimensional feature map in the alignment direction in which the images are aligned in the scanning direction.

Therefore, a two-dimensional feature map can be output that reflects the spatial characteristics of the three-dimensional structure with the images aligned in the scanning direction of the detection target.

The image alignment unit 173 is not limited to aligning the images in the order of the scanning direction, and may, for example, align the images in coordinate order in a predetermined direction.

In the fourth embodiment, the image processing apparatus 103 is also applicable to real-time detection tasks for ultrasound medical images. That is, the image alignment unit 173 can select a plurality of image frames from those including the current frame and several previous frames, and align these frames not in chronological order but in the order of the scanning direction, for example.

In the image processing apparatus 103 of the fourth embodiment, by further aligning the image queue in the order of the scanning direction, it is possible to output a two-dimensional feature map that more faithfully reflects the spatial characteristics of the three-dimensional structure of the detection target. Accordingly, by using this two-dimensional feature map in the detection task, detection accuracy can be further improved.

In the above-described embodiments, a plurality of functional units included in the image processing apparatus may be implemented as software on devices provided with a processor and a memory, such as independent computers, or may be implemented in a distributed manner across a plurality of devices, and may be achieved by the processor executing the functional units of an image processing apparatus stored in the memory. Each function of the image processing apparatus may also be implemented in hardware as circuits capable of executing the functions. A circuit that implements the image processing apparatus is capable of transmitting and receiving data, as well as collecting data, via a network such as the Internet. The functions of the respective units of the image processing apparatus may be implemented as a computer program product by having a processor of a computer execute a computer program previously stored in a recording medium.

In the above-described embodiments, the image processing apparatus is not limited to being implemented by a single processor, and may be configured by combining a plurality of independent processors, with each processor executing a computer program to achieve processing functions. Each function of the image processing apparatus may be appropriately distributed among or integrated into one or more processing circuits for implementation. Each function of the image processing apparatus may also be implemented by a combination of hardware, such as circuits and software. Computer programs corresponding to the respective functions of the image processing apparatus may be stored in a single memory circuit, or the computer programs corresponding to the respective functions may be distributed and stored across a plurality of memory circuits, with the image processing apparatus reading and executing each program from each memory circuit.

The term "processor" used in the image processing apparatus means, for example, a circuitry such as a central processing unit (CPU), a graphics processing unit (GPU), an application specific integrated circuit (ASIC), or a programmable logic device (for example, simple programmable logic device (SPLD), complex programmable logic device (CPLD), and field programmable gate array (FPGA)). Here, instead of storing the computer programs in the memory circuit, the computer programs may be configured to be incorporated directly into the circuit of the processor. In this case, the processor reads and executes the computer programs incorporated in the circuit to execute the functions thereof. Each processor of the present embodiment is not limited to the configuration of a single circuit provided for each processor, and may also employ the configuration of a single processor including a plurality of independent circuits in combination to execute the functions thereof.

The computer program product for implementing the functions of the respective units of the image processing apparatus includes a computer program executed by a processor, and this computer program is embedded in a read only memory (ROM) or a memory circuit in advance and then provided. These computer programs may be stored and provided in formats that can be installed in these apparatuses or as files in executable formats in a non-transitory computer readable medium, such as a compact disc (CD)-ROM, flexible Disk (FD), CD-recordable (R), digital versatile disc (DVD), or other storage medium. These programs may also be stored in a computer connected to a network, such as the Internet, and may be provided or distributed by downloading via the network. For example, these computer programs include modules that have processing functions described above. As for the actual hardware, the CPU reads and executes the computer programs from a storage medium such as a ROM, and each module is loaded in a main memory device and generated in the main memory device.

Each of the components of each of the apparatuses illustrated in the above-described embodiments is a functional concept, and does not necessarily have the physical configuration as illustrated in the figures. That is, the specific form of distribution and integration of each of the apparatuses is not limited to those illustrated in the figures, and all or parts thereof can be functionally or physically distributed and integrated in any units according to various loads and usage conditions. Furthermore, each of the functions performed by each of the apparatuses can be implemented, in all or any parts thereof, by CPU and a computer program analyzed and executed by the CPU, or by hardware using wired logic.

The various types of data handled herein are typically digital data.

According to at least one of the embodiments described above, it is possible to reflect spatial characteristics of the three-dimensional structure of a detection target.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the inventions as defined by the appended claims. The accompanying claims are intended to cover such forms or modifications as would fall within the scope of the invention as defined by the appended claims.

**Claims**

1. A medical image processing apparatus (102) comprising:

   an image acquisition unit (112) configured to acquire a plurality of medical images;
   a spatial information acquisition unit (152) configured to acquire three-dimensional spatial information corresponding to the medical images and representing three-dimensional spatial characteristics;
   a feature map generation unit (122, 132) configured to generate a feature map based on the medical images and the three-dimensional spatial information; and
   an execution unit (162) configured to execute processing on a target medical image based on the feature map.

2. The medical image processing apparatus (102) according to claim 1, wherein

   the feature map generation unit (122, 132) is further configured to generate a three-dimensional feature map based on the medical images and the three-dimensional spatial information, and generate a two-dimensional feature map by compressing the three-dimensional feature map, and

the execution unit (162) is further configured to execute processing on the target medical image based on the two-dimensional feature map.

3. The medical image processing apparatus (102) according to claim 2, wherein

the image acquisition unit (112) is further configured to acquire a plurality of medical images in a time series, and the feature map generation unit (122, 132) is further configured to generate the two-dimensional feature map by compressing the three-dimensional feature map in a time-series direction.

4. The medical image processing apparatus (102) according to claim 3, wherein
the feature map generation unit (122, 132) is further configured to construct a three-dimensional image by aligning the medical images in a chronological order, and generate the three-dimensional feature map based on the three-dimensional image.

5. The medical image processing apparatus (102) according to claim 4, wherein
the feature map generation unit (122, 132) is further configured to construct the three-dimensional image using smaller than a predetermined number of images among the medical images.

6. The medical image processing apparatus (102) according to claim 4, wherein

the spatial information acquisition unit (152) is further configured to acquire, as the three-dimensional spatial information, a similarity between images included in the medical images, and
the feature map generation unit (122, 132) is further configured to construct the three-dimensional image using a medical image whose similarity is smaller than a threshold value, among the medical images.

7. The medical image processing apparatus (102) according to claim 6, wherein

the spatial information acquisition unit (152) is further configured to acquire, as the similarity, a value representing an error between the images included in the medical images, and
the feature map generation unit (122, 132) is further configured to construct the three-dimensional image using, as the medical image whose similarity is smaller than a threshold value, a medical image whose value representing the error between the images is greater than a threshold value.

8. The medical image processing apparatus (102) according to claim 6, wherein

the spatial information acquisition unit (152) is further configured to acquire, as the similarity, a value representing a distance between the images included in the medical images based on spatial coordinates corresponding to each of the medical images, and
the feature map generation unit (122, 132) is further configured to construct the three-dimensional image using, as the medical image whose similarity is less than a threshold value, a medical image whose value representing the distance between the images is greater than a threshold value.

9. The medical image processing apparatus (102) according to claim 4, wherein
the feature map generation unit (122, 132) is further configured to generate the three-dimensional feature map by applying three-dimensional convolution to the three-dimensional image using a convolutional neural network model.

10. The medical image processing apparatus (102) according to claim 3, wherein
the feature map generation unit (122, 132) is further configured to compress the three-dimensional feature map in the time-series direction by averaging the three-dimensional feature map in the time-series direction.

11. The medical image processing apparatus (102) according to claim 3, wherein
the feature map generation unit (122, 132) is further configured to assign weights to the three-dimensional feature map at individual positions in the time-series direction, and compress the three-dimensional feature map in accordance with the assigned weight to generate the two-dimensional feature map.

12. The medical image processing apparatus (102) according to claim 11, wherein
the feature map generation unit (122, 132) is further configured to assign greater weights to positions closer to the current frame in the time-series direction with respect to the three-dimensional feature map.

13. An ultrasound diagnostic apparatus (1000) comprising:

an ultrasound probe (200) capable of imaging a subject; and
the medical image processing apparatus (102) according to any one of claims 1 to 12, wherein
the image acquisition unit (112) is further configured to acquire, as the plurality of medical images, a plurality of ultrasound medical images in a time series sequentially imaged by the ultrasound probe,
the spatial information acquisition unit (152) is further configured to acquire, as the three-dimensional spatial information, three-dimensional spatial information corresponding to the ultrasound medical images and representing three-dimensional spatial characteristics,
the feature map generation unit (122, 132) is further configured to generate, as the feature map, a feature map based on the ultrasound medical images and the three-dimensional spatial information, and
the execution unit (162) is further configured to execute, as the processing, processing on a target ultrasound medical image based on the feature map.

14. A medical image processing method comprising:

a step of acquiring a plurality of medical images;
a step of acquiring three-dimensional spatial information corresponding to the medical images and representing three-dimensional spatial characteristics;
a step of generating a feature map based on the medical images and the three-dimensional spatial information; and
a step of executing processing on a target medical image based on the feature map.

15. A computer readable medium comprising instructions that cause a computer to execute:

acquiring a plurality of medical images;
acquiring three-dimensional spatial information corresponding to the medical images and representing three-dimensional spatial characteristics;
generating a feature map based on the medical images and the three-dimensional spatial information; and
executing processing on a target medical image based on the feature map.

# FIG.1

IMAGE PROCESSING APPARATUS $\lceil$100

IMAGE ACQUISITION UNIT $\lceil$110

THREE-DIMENSIONAL FEATURE MAP GENERATION UNIT $\lceil$120

TWO-DIMENSIONAL FEATURE MAP GENERATION UNIT $\lceil$130

OUTPUT UNIT $\lceil$140

# FIG.2

IMAGE QUEUE

THREE-DIMENSIONAL
FEATURE EXTRACTION
(THREE-DIMENSIONAL
CONVOLUTION)

THREE-DIMENSIONAL
FEATURE MAP
$F_{3D} = f(l, w, h, c)$

$L_0 \times W_0 \times H_0 \times 3$

$L_1 \times W_1 \times H_1 \times C_1$

# FIG.3

THREE-DIMENSIONAL
FEATURE MAP
$F_{3D} = f(l, w, h, c)$

FEATURE
COMPRESSION

TWO-DIMENSIONAL
FEATURE MAP

$L_1 \times W_1 \times H_1 \times C_1$

$W_1 \times H_1 \times C_1$

# FIG.4

START

ACQUIRE PLURALITY OF IMAGES — S110

CONSTRUCT THREE-DIMENSIONAL IMAGE TO GENERATE THREE-DIMENSIONAL FEATURE MAP — S120

GENERATE TWO-DIMENSIONAL FEATURE MAP BY COMPRESSING THREE-DIMENSIONAL FEATURE MAP — S130

OUTPUT TWO-DIMENSIONAL FEATURE MAP — S140

END

# FIG.5

**IMAGE PROCESSING APPARATUS** ⌐101

**IMAGE ACQUISITION UNIT** ⌐111

**THREE-DIMENSIONAL FEATURE MAP GENERATION UNIT** ⌐121

**TWO-DIMENSIONAL FEATURE MAP GENERATION UNIT** ⌐131

**OUTPUT UNIT** ⌐141

**IMAGE SELECTION UNIT** ⌐151

# FIG.6

**IMAGE SELECTION UNIT** ⌐151

**CURRENT FRAME MANAGEMENT MODULE** ⌐1511

**IMAGE QUEUE MANAGEMENT MODULE** ⌐1512

**SIMILARITY MODULE** ⌐1513

**OUTPUT MODULE** ⌐1514

**SPATIAL COORDINATE ACQUISITION MODULE** ⌐1515

EP 4 776 221 A1

# FIG.7

START

HAS FRAME
BEEN ACQUIRED?  S111

NO

YES

UPDATE CURRENT FRAME  S112

WHETHER
OR NOT TO ALLOW
CURRENT FRAME TO BE
ADDED INTO IMAGE
QUEUE?  S113

NO

YES

IS
QUEUE LENGTH
GREATER THAN THRESHOLD
VALUE?  S114

YES

REMOVE LEADING
FRAME IN IMAGE QUEUE  S115

NO

S120

# FIG.8

S112

ACQUIRE REARMOST
FRAME IN IMAGE QUEUE — S1131

↓

CALCULATE SIMILARITY BETWEEN
CURRENT FRAME AND REARMOST
FRAME — S1132

↓

IS SIMILARITY
SMALLER THAN THRESHOLD
VALUE? — S1133 → NO

YES

UPDATE REARMOST FRAME — S1134

↓

S113: YES

S113: NO

# FIG.9

102

IMAGE PROCESSING APPARATUS

112
IMAGE ACQUISITION UNIT

122
THREE-DIMENSIONAL FEATURE MAP GENERATION UNIT

132
TWO-DIMENSIONAL FEATURE MAP GENERATION UNIT

142
OUTPUT UNIT

152
IMAGE SELECTION UNIT

162
DETECTION UNIT

# FIG.10

162

DETECTION UNIT

1621
TARGET EXTRACTION MODULE

1622
IMAGE SEGMENTATION MODULE

1623
IMAGE CLASSIFICATION MODULE

# FIG.11

ULTRASOUND DIAGNOSTIC APPARATUS — 1000

IMAGE PROCESSING APPARATUS — 102

ULTRASOUND PROBE — 200

IMAGE ACQUISITION UNIT — 112

THREE-DIMENSIONAL FEATURE MAP GENERATION UNIT — 122

TWO-DIMENSIONAL FEATURE MAP GENERATION UNIT — 132

OUTPUT UNIT — 142

IMAGE SELECTION UNIT — 152

DETECTION UNIT — 162

DISPLAY — 300

EP 4 776 221 A1

# FIG.12

## FIG.13

## FIG.14

# FIG.15

IMAGE PROCESSING APPARATUS — 103

IMAGE ACQUISITION UNIT — 113

THREE-DIMENSIONAL FEATURE MAP GENERATION UNIT — 123

TWO-DIMENSIONAL FEATURE MAP GENERATION UNIT — 133

OUTPUT UNIT — 143

IMAGE ALIGNMENT UNIT — 173

Europäisches
Patentamt

European
Patent Office

Office européen
des brevets

# EUROPEAN SEARCH REPORT

**Application Number**

EP 26 15 0822

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 112 116 562 A (CHONGQING ZHONG DI MEDICAL INFORMATION TECH CO LTD) 22 December 2020 (2020-12-22) | 1-4,9-15 | INV. G06T7/00 A61B8/00 |
| A | * paragraphs [0039] - [0047]; figure 1 * ----- | 5-8 | |
| A | US 2021/049397 A1 (CHEN SIHONG [CN]) 18 February 2021 (2021-02-18) * the whole document * ----- | 1-15 | |
| A | US 2019/130578 A1 (GULSUN MEHMET AKIF [US] ET AL) 2 May 2019 (2019-05-02) * the whole document * ----- | 1-15 | |

**TECHNICAL FIELDS SEARCHED (IPC)**

G06T
A61B

The present search report has been drawn up for all claims

| Place of search | Date of completion of the search | Examiner |
|---|---|---|
| Munich | 19 May 2026 | Tillier, Christophe |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 26 15 0822

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2026

| Patent document cited in search report | | Publication date | Patent family member(s) | | Publication date |
|---|---|---|---|---|---|
| CN 112116562 | A | 22-12-2020 | NONE | | |
| US 2021049397 | A1 | 18-02-2021 | CN | 109446951 A | 08-03-2019 |
| | | | CN | 111126242 A | 08-05-2020 |
| | | | EP | 3869387 A1 | 25-08-2021 |
| | | | US | 2021049397 A1 | 18-02-2021 |
| | | | WO | 2020078269 A1 | 23-04-2020 |
| US 2019130578 | A1 | 02-05-2019 | NONE | | |

EPO FORM P0459

For more details about this annex : see Official Journal of the European Patent Office, No. 12/82